# EUROPÄISCHE PATENTANMELDUNG

(11) **EP 4 445 874 A2**
(43) Veröffentlichungstag der Anmeldung: **16.10.2024**
(21) Anmeldenummer: 24198102.6
(22) Anmeldetag: 20.05.2020
(51) Int. Cl.: A61F 2/32

(54) **HÜFTGELENKIMPLANTAT MIT NACHFORMBAREN BEFESTIGUNGSLASCHEN**

(30) Priorität: 24.05.2019 EP 19176517
(62) Teilanmeldung aus: 20726152.0
(71) Anmelder: Waldemar Link GmbH & Co. KG, 22339 Hamburg (DE)
(72) Erfinder: Link, Helmut D., 22397 Hamburg (DE); Fischer, Hans-Joachim, 22850 Norderstedt (DE)
(74) Vertreter: Glawe, Delfs, Moll

(57) **Zusammenfassung**

Hüftgelenkimplantat zum Befestigen an einem Beckenknochen (9) mit einem eine Pfanne (6) aufweisenden Tragkörper, dessen konvexe Außenfläche zur Anlage an den Beckenknochen (9) ausgebildet ist und an seiner konkaven Innenseite eine Aufnahme für ein beckenseitiges Lager aufweist, das zur Aufnahme eines Gelenkkopfs einer Femurkomponente einer Hüftprothese ausgebildet ist, und am Randbereich der Pfanne (6) angeordneten nach außen weisenden, flachen Befestigungslaschen (2, 3), die jeweils mit mindestens einer Aufnahme für ein Befestigungsmittel versehen sind. Die Befestigungslaschen (2, 3) bestehen aus einem nachformbaren biokompatiblen Material und sind über eine unlösbare stoffschlüssige Verbindung (7) mit der Pfanne (6) verbunden, wobei die Pfanne (6) aus einem anderen, steiferen biokompatiblen Material besteht. Eine hohe Robustheit der Pfanne (6) wird so kombiniert mit einer dank Nachverformbarkeit verbesserten Anpassung an die anatomischen Verhältnisse des jeweiligen Beckenknochens. Befestigungssicherheit, Stabilität und Langzeitverhalten werden so verbessert.

## Beschreibung

Die Erfindung betrifft ein Hüftgelenkimplantat zum Befestigen an einem Beckenknochen, mit einem eine Pfanne aufweisenden Tragkörper, dessen konvexe Außenfläche zur Anlage an den Beckenknochen ausgebildet ist und an seiner konkaven Innenseite eine Aufnahme für beckenseitige Lagerkomponenten aufweist, die zur Aufnahme eines Gelenkkopfs einer Femurkomponente einer Hüftprothese ausgebildet ist, und am Randbereich der Pfanne angeordneten nach außen weisenden, flachen Befestigungslaschen, die jeweils mit mindestens einer Aufnahme für ein Befestigungsmittel versehen sind. Sie betrifft ferner ein Herstellungsverfahren für solche Hüftgelenkimplantate.

Hüftgelenke können durch Krankheit oder durch Verschleiß degeneriert sein. Zur Therapie sind Hüftgelenk-Endoprothesen bewährt. Sie umfassen eine Femurkomponente, die am oberen Ende des Oberschenkelknochens implantiert wird und eine damit zusammenwirkende Hüft-Komponente, die im Beckenknochen (Acetabulum) implantiert wird. In vielen Fällen kann es vorkommen, dass der Beckenknochen geschädigt ist im Bereich des Hüftgelenks. Dies hat zur Folge, dass die Hüftgelenk-Komponente schwierig an Beckenknochen zu befestigen ist. Dies gilt insbesondere für solche Implantate, die als Becken-Teilersatz fungieren. Diese weisen zusätzliche Befestigungselemente auf, um nicht nur eine sichere Verankerung der Gelenkspfanne des Hüftgelenkimplantats am Beckenknochen zu erreichen, sondern darüber hinaus auch den Beckenknochen in sich zu stabilisieren.

Derartige Hüftgelenkimplantate sind beispielsweise bekannt aus der US 5 702 477. Sie weisen an einem die eigentliche Gelenkpfanne umfassenden Tragkörper mehrere Befestigungselemente auf. Diese umfassen sich nach kranial erstreckende längliche Laschen, die mit einer Mehrzahl von Bohrlöchern zur Befestigung mittels Knochenschraube versehen sind. Ferner ist gegenüberliegend eine nach kaudal gerichtete kürzere Lasche vorgesehen, die ggf. auch hakenartig ausgeführt sein kann. Sie dienen zur sicheren Befestigung und Stabilisierung des Implantats, und insbesondere von dessen Gelenkpfanne, an einem beschädigten Beckenknochen.

Hüftgelenkimplantate mit solchen Befestigungslaschen werden als Becken-Teilersatz auch von der Anmelderin hergestellt und vertrieben. Aus Gründen kostengünstiger Herstellung und hoher mechanischer Robustheit wird als Material rostfreier Stahl (Implantatstahl) gewählt. Dieser bietet den Vorzug, dass zum einen der Tragkörper mit der Pfanne hinreichend robust ist und eine ausreichende mechanische Stabilität aufweist, und andererseits die Befestigungslaschen je nach individueller Anatomie des Patienten und des zu überbrückenden Knochendefekts noch intraoperativ durch Nachformen ohne weiteres angepasst werden können. Obgleich somit funktional eine befriedigende Lösung erreicht ist, besteht die Schwierigkeit, dass aus Gründen besserer Biokompatibilität häufig Titan als Material bevorzugt ist. Bewährt für Implantate ist TiAl6V4, das zwar hinreichend robust ist, aber den Nachteil aufweist, nicht intraoperativ nachformbar zu sein. Reintitan hingegen ist deutlich einfacher nachformbar, wird aber mangels Formstabilität standardmäßig nicht für die beckenseitige Pfannenkomponente eingesetzt. Dieser Zielkonflikt konnte bisher nicht befriedigend aufgelöst werden.

Der Erfindung liegt die Aufgabe zugrunde, ein verbessertes Hüftgelenkimplantat zu schaffen, das diese Nachteile vermeidet. Die Erfindung erstreckt sich ferner auf ein entsprechendes Herstellungsverfahren.

Die erfindungsgemäße Lösung liegt in den Merkmalen der unabhängigen Ansprüche. Vorteilhafte Weiterbildungen sind Gegenstand der abhängigen Ansprüche.

Bei einem Hüftgelenkimplantat zum Befestigen an einem Beckenknochen, mit einem eine Pfanne aufweisenden Tragkörper, dessen konvexe Außenfläche zur Anlage an den Beckenknochen ausgebildet ist und an seiner konkaven Innenseite eine Aufnahme für eine beckenseitige Lagerkomponente aufweist, die zur Aufnahme eines Gelenkkopfs einer Femurkomponente einer Hüftprothese ausgebildet ist, und am Randbereich der Pfanne angeordneten nach außen weisenden, flachen Befestigungslaschen, die jeweils mit mindestens einer Aufnahme für ein Befestigungsmittel versehen sind, ist erfindungsgemäß vorgesehen, dass die Befestigungslaschen aus einem nachformbaren (kaltformbaren) biokompatiblen Material bestehen und über eine unlösbare stoffschlüssige Verbindung mit der Pfanne verbunden sind, wobei die Pfanne aus einem anderen, steiferen biokompatiblen Material besteht.

Nachfolgend seien zuerst einige verwendete Begriffe erläutert:
Unter "nachformbar" wird ein bei Raumtemperatur derart plastisch verformbares Material verstanden, dass aus diesem Material gebildete Befestigungslaschen manuell mit oder ohne Handwerkzeuge an eine unterliegende Struktur angepasst werden können. Dies wird auch als kaltverformbar bezeichnet.

Im Gegensatz dazu wird als nicht kaltverformbar solches Material bezeichnet, welches bei Raumtemperatur manuell oder mittels Handwerkzeugen nicht plastisch verformt werden kann.

Unter biokompatibel wird ein Material verstanden, dass zugelassen und geeignet ist für die Herstellung von Implantaten und Endoprothesen.

Die Lagerkomponente kann als ein Einsatz aus gleitgünstigem Material ausgeführt sein, der als Aufnahme und Gleitpartner für einen Gelenkkopf fungiert. Üblicherweise wird solch ein Einsatz zementiert oder verklemmt in dem Tragkörper befestigt, es können aber auch andere Befestigungsarten vorgesehen sein. Die Lagerkomponente kann aber auch als eine Interpositionskomponente ausgeführt sein. Hierunter versteht man üblicherweise ein solches Zwischenstück, das einen Öffnungswinkel der Pfanne verändert, insbesondere zur Anpassung an spezielle anatomische Verhältnisse. In diesem ist dann das den Gleitpartner für den Gelenkkopf bildende Element aufgenommen. Ferner kann die Lagerkomponente auch als sog. Dual-Mobility-Einsatz in den Tragkörper eingebracht und fixiert sein.

Die Erfindung basiert auf dem Gedanken, eine Materialaufteilung in dem Sinne vorzunehmen, dass für die Befestigungslaschen und den pfannenartigen Tragkörper verschiedene Materialien verwendet sind, die stoffschlüssig und damit unlösbar miteinander verbunden sind. Die Befestigungslaschen sind hergestellt aus einem nachformbaren biokompatiblen Material, beispielsweise Reintitan (Grad 2, Grad 3 oder Grad 4). Dieses Material ist für den Chirurgen gut auch intraoperativ nachverformbar, so dass er eine gute Anpassung an die jeweiligen individuellen Besonderheiten des Beckenknochens des jeweiligen Patienten problemlos erreichen kann. Für den Tragkörper mit der Pfanne, die mittels einer Lagerkomponente das Lager für den Gelenkkopf der Femurkomponente bildet, wird hingegen auf ein anderes biokompatibles Material zurückgegriffen, beispielsweise eine Standard-Titanlegierung wie TiAl6V4. Dieses Material ist steifer, kostengünstig, mechanisch robust und nur bedingt nachverformbar. Für die Pfanne ist letzteres aber auch gar nicht erforderlich; im Gegenteil, die mangelnde Nachverformbarkeit erhöht die Robustheit und Maßhaltigkeit der Pfanne, und zwar auch unter hohen Lasten. Dank der Maßhaltigkeit eignet sich die erfindungsgemäße Konstruktion besonders gut für Pfannen in Modulbauweise, wobei ggf. alternative Pfannen und Pfanneneinsätze verwendet werden.

Die Erfindung hat erkannt, dass auf diese Weise der bisher nicht gelöste Widerspruch zwischen robustem und kostengünstigem Implantat einerseits und guter Nachverformbarkeit zur besseren Anpassung und Befestigung an die individuelle Form des Beckenknochens andererseits gelöst werden kann. Dank der stoffschlüssigen Verbindung zwischen den Befestigungslaschen und der Pfanne erreicht sie dies ohne Einbußen in Bezug auf die Befestigungssicherheit, Stabilität und Langzeitfestigkeit des Hüftgelenkimplantats. Die diesbezüglichen Nachteile einer mehrteiligen, verschraubten Konstruktion, wie aus DE 41 02 510 A1 bekannt, werden so wirksam vermieden. Außerdem wird zusätzlich mit der stoffschlüssigen Verbindung nicht nur eine Steigerung der Zuverlässigkeit, sondern auch eine Bauraumersparnis gegenüber der bekannten Verschraubung erreicht.

Die stoffschlüssige Verbindung ist zweckmäßigerweise als eine Schweißverbindung ausgeführt, und zwar insbesondere als eine Elektronenstrahl-Schweißverbindung. Dies bietet den Vorteil hoher Reproduzierbarkeit und eines hervorragenden Schweißergebnisses bei minimalem Wärmeeintrag. Als Folge des geringen Wärmeeintrags entsteht auch nur ein äußerst geringer Verzug. Ferner ermöglicht es die Verschweißung verschiedener Werkstoffe, insbesondere von (Rein)Titan mit Titanlegierungen. Schließlich erlaubt diese Verbindung einen hohen Automatisierungsgrad, was ebenfalls der Reproduzierbarkeit und damit Güte der Verbindung insgesamt zugutekommt.

Mit Vorteil ist die stoffschlüssige Verbindung ausgeführt mit einer durchgeschweißten Schweißnaht. Besonders zweckmäßig ist eine sog. i-Naht, die eine zuverlässige Befestigung und ein sicheres Durchschweißen bei geringem Wärmeeintrag ermöglicht.

Die Befestigungslaschen können grundsätzlich zwar einzeln ausgeführt sein. Besonders bevorzugt ist es jedoch im Rahmen der vorliegenden Erfindung, die Befestigungslaschen einteilig mit einem Befestigungsring auszuführen, der den pfannenartigen Tragkörper umgreift und mittels der stoffschlüssigen Verbindung mit der Pfanne verbunden ist. Auf diese Weise ergibt sich eine gute Vormontierbarkeit, nämlich von den Befestigungslaschen mit dem Befestigungsring. Nachdem dieser vorgefertigt (und gegebenenfalls nachbearbeitet) ist, kann er mit dem pfannenartigen Tragkörper vormontiert werden. Die Vormontage ermöglicht eine rationelle Bereitstellung, bevor die Komponenten zum Schweißen gelangen. Um eine hohe Positionierungsgenauigkeit zu gewährleisten, umfasst vorzugsweise das Vormontieren ein Verpressen von pfannenartigem Tragkörper mit dem Befestigungsring und den daran angeordneten Befestigungslaschen.

Die Befestigungslaschen können vorgefertigt sein mit Öffnungen für Befestigungsmittel. In den meisten Fällen werden diese Bohrungen sein, die vorbereitet sind zur Aufnahme von Befestigungsschrauben (Knochenschrauben) zur Befestigung der Lasche am Beckenknochen. Ferner kann der Befestigungsring insoweit vorgefertigt sein, dass er eine Öffnung aufweist, die so bemessen ist, dass sie zur Aufnahme des pfannenartigen Tragkörpers fungiert. Die Öffnung ist vorzugsweise maßgenau auf eine Außenabmessung des Tragkörpers vorgefertigt, und zwar insbesondere in Bezug auf einen Außendurchmesser der Pfanne des Tragkörpers. Besonders vorteilhaft ist eine Ausführung der Öffnung im Befestigungsring derart, dass sie eine Presspassung mit dem pfannenartigen Tragkörper bildet. Dies ermöglicht eine initiale Fixierung, welche die Vormontierbarkeit begünstigt.

Die Pfanne selber kann aus einem nicht kaltverformbaren Material bestehen, beispielsweise aus einer Titanlegierung, die weniger verformbar ist als Reintitan. Bewährt haben sich hierfür beispielsweise eine Legierung aus TiAl6V4, die eine Zugfestigkeit von 800 MPa oder größer erreicht, wobei Reintitan nur eine Zugfestigkeit von ca. 300 MPa erzielt. Für die Befestigungslaschen bzw. den Befestigungsring hat sich Reintitan bewährt, insbesondere Reintitan nach Grad 2, Grad 3 oder Grad 4. Es verknüpft eine hohe Biokompatibilität mit guter Nachverformbarkeit.

Die Pfanne ist vorzugsweise im Bereich der Aufnahme des Befestigungsrings mit einer Stützschulter und/oder einem Zentrierring versehen. Die Stützschulter ist zweckmäßigerweise umlaufend ausgeführt. Damit kann sie einen Anschlag für den Befestigungsring bilden, sodass dessen Position beim Aufschieben des Befestigungsrings auf die Pfanne definiert ist. Der Zentrierring sichert eine genau zentrale Lage. Dies kann auch als Schnittstelle für ein Modulsystem dienen. Dies ermöglicht es, auch andere Arten von Pfannen einzusetzen, beispielsweise auch solche Pfannen, die zu einem aus mehreren Komponenten bestehenden Modulsystem gehören. Dazu können auch solche Pfannen gehören, die verschieden gestaltet sind aber einen einheitlichen Außendurchmesser aufweisen. Dies gilt insbesondere dann, wenn mehrere einheitliche Außendurchmesser vorgesehen sind, die in verschiedenen Außendurchmesser-Größen abgestuft sind.

Der Übergangsbereich zwischen Pfanne und Befestigungslaschen bzw. Befestigungsring kann mit einer gefrästen Fläche versehen sein. Diese kann beispielsweise aufgebracht werden im Rahmen einer Nachbearbeitung nach der stoffschlüssigen Verbindung. Damit wird zum einen eine Verkleinerung insbesondere des Befestigungsrings erzielt und zum anderen eine Verbesserung der Oberflächengüte erreicht.

Die Befestigungslaschen können vorgeformt sein. Dies bietet den Vorteil, dass bereits so eine Grobanpassung an die typische anatomische Struktur des Beckenknochens erreicht werden kann. Der Operateur braucht lediglich noch zur Feinanpassung die Laschen entsprechend nachzubiegen. Damit ergibt sich eine erhebliche Arbeitsersparnis.

Die Erfindung bezieht sich ferner auf ein Verfahren zur Herstellung eines entsprechenden Hüftgelenkimplantats.

Zur näheren Erläuterung wird auf vorstehende Beschreibung verwiesen.

Die Erfindung wird nachfolgend unter Bezugnahme auf die beigefügte Zeichnung anhand eines Ausführungsbeispiels näher erläutert. Es zeigen:
- Fig. 1: ein Ausführungsbeispiel des Hüftgelenkimplantats im eingebauten Zustand an einen Beckenknochen;
- Fig. 2: eine Ansicht eines vorgefertigten Befestigungsrings mit Befestigungslaschen;
- Fig. 3: eine perspektivische Ansicht eines pfannenartigen Tragkörpers;
- Fig. 4: eine perspektivische Ansicht eines vormontierten Zustands;
- Fig. 5: eine Querschnittsansicht zu einer Schweißnaht als Verbindung zwischen Befestigungsring/-laschen und Tragkörper;
- Fig. 6: eine perspektivische Ansicht eines fertiggestellten Hüftgelenkimplantats, mit gebogenen Befestigungslaschen und mit montagebereiter Lagerkomponente; und
- Fig. 7a, b: eine Ausführung des pfannenartigen Tragkörpers in Modulbauweise.

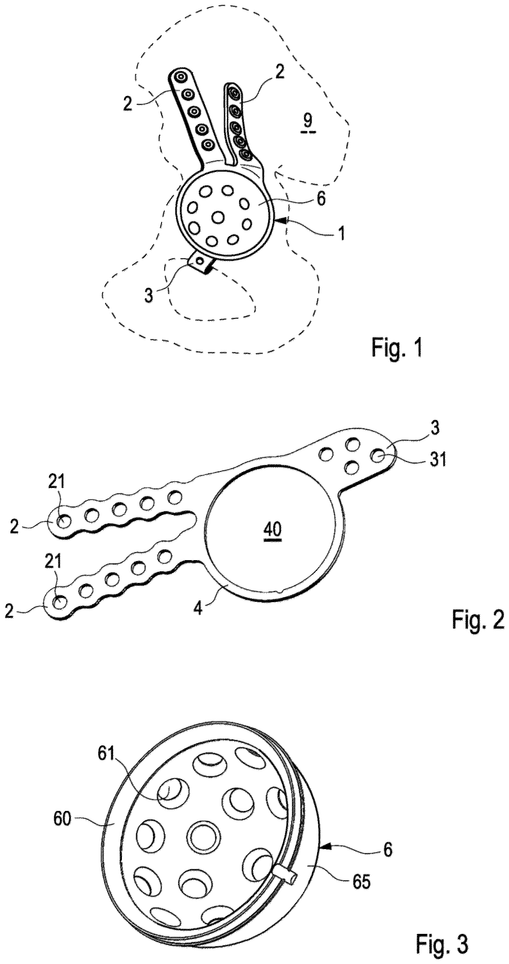

Ein Ausführungsbeispiel für ein Hüftgelenksimplantat gemäß der Erfindung ist in Figur 1 in einem an einem Beckenknochen 9 (Acetabulum) eingesetzten Zustand dargestellt.

Das Hüftgelenkimplantat ist in seiner Gesamtheit mit der Bezugsziffer 1 bezeichnet und umfasst einen pfannenartigen Tragkörper 6, an dem nach oben weisend (kranial) zwei flache und langgestreckte Befestigungslaschen 2 angeordnet sind. An dem pfannenartigen Tragkörper 6 (im Folgenden auch kurz: Pfanne) gegenüberliegend ist eine kürzere, sich nach unten (kaudal) erstreckende Befestigungslaschen 3 vorgesehen.

In dem dargestellten Ausführungsbeispiel besteht das Implantat 1 aus zwei Komponenten, der Pfanne 6 sowie einer Befestigungskomponente, welche die Befestigungslaschen 2, 3 sowie einen Befestigungsring 4 umfasst, an dem die Befestigungslaschen 2, 3 angeordnet sind. Die Befestigungslaschen 2 sind jeweils lang gestreckte flache Elemente, die mit einer Vielzahl von Bohrungen 21 zur Aufnahme von Befestigungsmittel versehen sind. Die Befestigungslasche 3 ist ebenfalls ein flaches Element; in dem dargestellten Ausführungsbeispiel jedoch kürzer und breiter ausgeführt als die Befestigungslaschen 2. Diese Ausführung ist lediglich beispielhaft; die Erfindung ist darauf nicht beschränkt. Die Befestigungslasche 3 ist ebenfalls mit einer Mehrzahl von Bohrungen 31 versehen zur Aufnahme von Befestigungsmitteln. Die Bohrungen 21, 31 sind als Durchgangsöffnungen ausgeführt und ausgebildet zur Aufnahme eines Befestigungsmittels (nicht dargestellt). Als Befestigungsmittel fungieren an sich bekannte Schrauben, insbesondere Knochenschrauben.

Es wird nun Bezug genommen auf Figur 2. Die Befestigungslaschen 2, 3 sind radial nach außen abstehend an einem Ring 4 angeordnet, der als Befestigungsring bezeichnet ist. In dem dargestellten Ausführungsbeispiel sind die Befestigungslaschen 2, 3 sowie der Befestigungsring 4 einstückig ausgeführt. Sie bestehen aus einem Titanmaterial, und zwar Reintitan von Grad 2 oder Grad 4. Dieses Material weist eine ausgesprochen gute Biokompatibilität auf und weist ferner den Vorzug auf, dass es auch gut manuell verformt werden kann. Es ist somit dem Operateur ermöglicht, die Befestigungslaschen 2, 3 auch noch während der Implantation, d. h. während der Operation, gegebenenfalls nachzuformen und so besser an die jeweiligen anatomischen Besonderheiten des Beckenknochens 9 des jeweiligen Patienten anzupassen. Die einstückige Ausführung des Befestigungsrings 4 sorgt für eine hohe Festigkeit und für eine gute Handhabung.

Der Befestigungsring 4 umrandet einen inneren Bereich 40, der zur Aufnahme des pfannenartigen Tragkörpers 6 ausgebildet ist. In dem dargestellten Ausführungsbeispiel ist der Befestigungsring 4 vollumschließend ausgeführt, unbedingt erforderlich ist dies nicht; der Ring braucht nicht vollständig geschlossen zu sein.

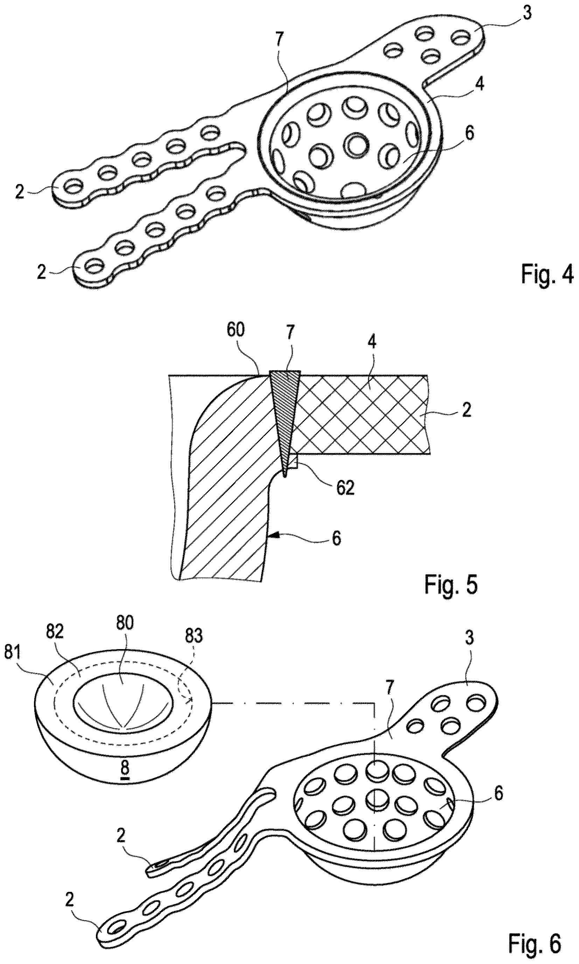

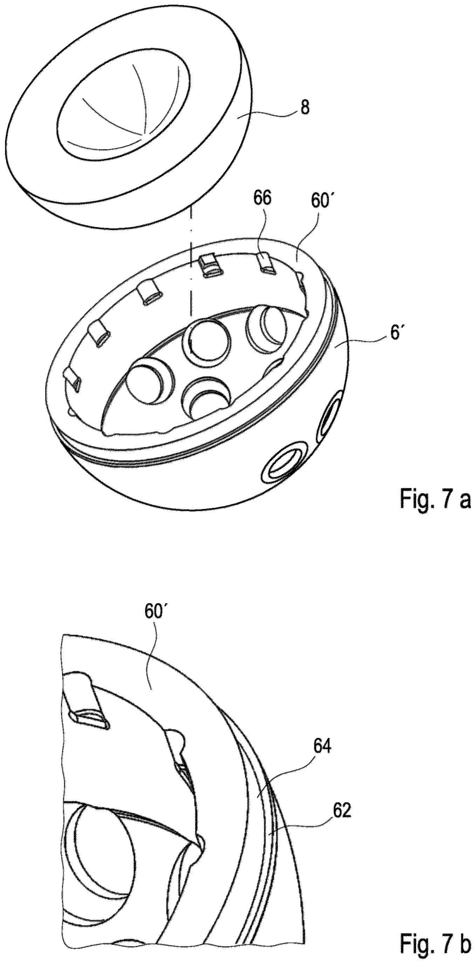

Der in Figur 3 dargestellte pfannenartige Tragkörper 6 weist eine im Wesentlichen halbkugelige Gestalt auf. Sein Hauptteil ist domartig gewölbt und mit einer Vielzahl von Befestigungsbohrungen 61 versehen. An seiner konvex geformten Außenfläche ist der pfannenartige Tragkörper 6 zur Anlage an die Knochenflächen des Beckenknochens 9 ausgebildet und dazu zweckmäßigerweise mit einer das Einwachsen von Knochenmaterial fördernden Oberfläche bzw. Beschichtung 65 versehen. An seiner Innenseite ist der pfannenartige Tragkörper entsprechend konkav gewölbt und bildet zusammen mit einer Lagerkomponente die Aufnahme für einen Gelenkkopf (nicht dargestellt) einer Femurkomponente von einer Hüftgelenkprothese. In die Pfanne 6 kann als Lagerkomponente eine aus gleitgünstigem Material (insbesondere Kunststoffmaterial) bestehende Lagerschale 8 (s. Fig. 6 bzw. 7) aufgenommen werden. Die Lagerschale 8 kann optional auch in einer Variante als sog. Dual-Mobility ausgeführt sein. Hierbei besteht die Lagerschale im Grunde aus zwei ineinander gesetzten Lagerschalen, einer Außenschale 81 und einer Innenschale 82. Man erhält so dazwischen eine weitere Lagerfläche 83 (in Fig. 6 durch gestrichelte Linie gezeigt). Der erreichte zusätzliche Freiheitsgrad verringert Verschleiß und Abrieb zwischen Lagerschale 8 und der Pfanne 6; ferner kann damit eine größere Beweglichkeit und damit ein verbesserter Schutz vor Luxation erreicht werden.

Der pfannenartige Tragkörper 6 besteht aus einer biokompatiblen Titanlegierung, die eine eher hohe Festigkeit aufweist zur Aufnahme der Lagerkräfte des Hüftgelenks. Bewährt hat sich als Titanlegierung insbesondere TiAl6V4. Es weist eine hohe Festigkeit und Robustheit auf. Typischerweise ist es zu steif, um von Hand verbogen zu werden. Es eignet sich somit besonders, um die von dem Gelenkkopf ausgeübten erheblichen Lagerkräfte des Hüftgelenks aufzunehmen und in den Beckenknochen 9 zu leiten.

Bei der Herstellung wird der pfannenartige Tragkörper 6 vormontiert, indem er in die Öffnung 40 des Befestigungrings 4 eingesetzt wird. Er wird dabei so weit eingesetzt, dass der Befestigungsring 4 im Bereich des oberen Endes am Pfannenrand 60 passgenau den pfannenartigen Tragkörper 6 umgreift, vorzugsweise nach Art einer Presspassung. Damit ist der pfannenartige Tragkörper 6 in einem vormontierten Zustand an dem Befestigungsring 4 mit Befestigungslaschen 2, 3 gehaltert, wie in Figur 4 dargestellt. In diesem Zustand kann dann durch Elektronenstrahl-Schweißen eine stoffschlüssige Verbindung 7 zwischen der Pfanne 6 und dem Befestigungsring 4 mit den Befestigungslaschen 2, 3 hergestellt werden. Vorzugsweise ist die stoffschlüssige Verbindung 7 ausgeführt mit einer durchgeschweißten Naht, insbesondere einer sogenannten "i-Naht", wie in Figur 5 dargestellt. Die Schweißverbindung 7 sichert eine feste Verbindung auch bei verschiedenen Werkstoffen, nämlich wie vorliegend zwischen der Titanlegierung TiAl6V4 für die Pfanne 6 und Reintitan (Grad 2 oder Grad 4) für die Befestigungslaschen 2, 3 mit ihrem Befestigungsring 4.

Ferner erkennt man in Figur 5 eine Stützschulter 62, welche eine Positionierung bzw. Abstützung des Befestigungrings 4 mit den daran angeordneten Befestigungslaschen 2, 3 an der Pfanne 6 erreicht. Also insbesondere eine planebene Anordnung erreicht, sodass sich oberseitig eine möglichst durchgehende Oberfläche bildet. Vorzugsweise kann weiter vorgesehen sein, nach dem Herstellen einer Schweißverbindung 7 durch Überfräsen einen glatten Übergang der Oberseite zwischen der Pfanne 6 mit ihrem Pfannenrand 60, der Schweißverbindung 7 und der Oberseite des Befestigungrings 4 zu schaffen. Dieser finale Zustand ist in Figur 6 dargestellt.

Schließlich können die Befestigungslaschen zumindest teilweise bereits vorgeformt werden. Dies ist ebenfalls in Figur 6 dargestellt. Hier sind die Befestigungslaschen 2 bereits vorgeformt an eine Kontur des Beckenknochens 9, wie sie typischerweise zu erwarten ist. Eine Feinanpassung an die jeweilige individuelle Anatomie des Beckenknochens kann dann durch den Operateur noch intraoperativ durch einfaches manuelles Verbiegen der Befestigungslaschen 2, 3 erfolgen.

Die Erfindung kann Anwendung finden im Rahmen eines modularen Hüftgelenksystems. Hierbei sind verschieden gestaltete Pfannen 6, 6' vorgesehen, die sich insbesondere im Hinblick auf ihre Größe (Dimensionierung) unterscheiden. So können Pfannen 6 in verschiedenen Größen vorgesehen sein, die jedoch in gleicher Weise mittels der Schweißverbindung 7 mit dem Befestigungsring 4 mit den Befestigungslaschen 2, 3 verbindbar sind. Die modularen Pfannen können sich aber auch in Bezug auf ihre weitere Gestaltung unterscheiden, insbesondere bei der Gestaltung der Innenoberfläche. Eine solche modular gestaltete Pfanne 6' ist in Figur 7a) dargestellt. Sie weist Positionierungsnuten 66 auf zur winkelgenauen Positionierung von modularen Lagereinsätzen (nicht dargestellt). In die Pfanne 6 kann eine Lagerschale 8 eingesetzt werden, wie schon vorstehend zu Figur 6 erläutert.

Optional kann an der Pfanne 6 ausgebildet sein ein Zentrierring 64, wie in Figur 7b) dargestellt. Er ist in Bezug auf seinen Außendurchmesser genau auf die Weite der Öffnung 40 abgestimmt und ermöglicht somit eine präzise Passung und Zentrierung der Pfanne 6 in Bezug auf den Befestigungsring 4 mit den Befestigungslaschen 2, 3. Besonders bei der Ausführung mit einer modularen Pfanne 6` kann mit einem Zentrierring 64 eine sichere Positionierung und Zentrierung auch von Pfannen verschiedener Größen erreicht werden. Der Zentrierring 64 kann als gesondertes Element ausgeführt sein oder, insbesondere bei Pfannen mit verhältnismäßig großer Dimensionierung, auch als Ausnehmung ausgebildet und somit einteilig mit der Pfanne 6 ausgeführt sein.

## Patentansprüche

1. Hüftgelenkimplantat (1) zum Befestigen an einem Beckenknochen (9) mit einem eine Pfanne (6) aufweisenden Tragkörper, dessen konvexe Außenfläche zur Anlage an den Beckenknochen (9) ausgebildet ist und an seiner konkaven Innenseite eine Aufnahme für ein beckenseitiges Lager aufweist, das zur Aufnahme eines Gelenkkopfs einer Femurkomponente einer Hüftprothese ausgebildet ist, und am Randbereich der Pfanne (6) angeordneten nach außen weisenden, flachen Befestigungslaschen (2, 3), die jeweils mit mindestens einer Aufnahme für ein Befestigungsmittel versehen sind,
**dadurch gekennzeichnet, dass**
die Befestigungslaschen (2, 3) einteilig aus biokompatiblen Material ausgeführt sind mit einem Befestigungsring (4), der vorgefertigt ist mit einer Öffnung zur Aufnahme (40) des pfannenartigen Tragkörpers (6), wobei der Befestigungsring (4) ausgebildet ist zur Verbindung mit der Pfanne (6), wobei der Befestigungsring (4) bemessen ist, um sich zu erstrecken um einen Umfang des Tragkörpers (6), und wobei eine Oberseite des Tragkörpers (6) und eine Oberseite des Befestigungsrings (4) eine flache, ebene Oberfläche bilden.

2. Hüftgelenkimplantat nach Anspruch 1, **dadurch gekennzeichnet, dass** die Befestigungslaschen (2, 3) aus einem nachformbaren biokompatiblen Material bestehen, und wobei die Pfanne (6) aus einem anderen, steiferen biokompatiblen Material besteht.

3. Hüftgelenkimplantat nach einem der vorangehenden Ansprüche, **dadurch gekennzeichnet, dass** der pfannenartige Tragkörper (6) im Bereich der Aufnahme des Befestigungsrings (4) mit einer vorzugsweise umlaufenden Stützschulter (62) versehen ist, so dass die Befestigungslaschen (2, 3) und der Befestigungsring (4) auf der Stützschulter (62) abgestützt sind.

4. Hüftgelenkimplantat nach Anspruch einem der vorangehenden Ansprüche, **dadurch gekennzeichnet, dass** die stoffschlüssige Verbindung (7) als eine Schweißverbindung, vorzugsweise eine Elektronenstrahl-Schweißverbindung, ausgeführt ist.

5. Hüftgelenkimplantat nach Anspruch 4, **dadurch gekennzeichnet, dass** die stoffschlüssige Verbindung (7) eine durchgeschweißte Schweißnaht aufweist.

6. Hüftgelenkimplantat nach einem der vorangehenden Ansprüche, **dadurch gekennzeichnet, dass** die Befestigungslaschen (2, 3) vorgefertigt sind mit Öffnungen (21, 31) für Befestigungsmittel.

7. Hüftgelenkimplantat nach einem der Ansprüche 1 bis 6, **dadurch gekennzeichnet, dass** die Aufnahme (40) maßgenau auf eine Außenabmessung des pfannenartigen Tragkörpers (6) vorgefertigt ist, vorzugsweise auf einen Außendurchmesser des pfannenartigen Tragkörpers (6), insbesondere als Presspassung.

8. Hüftgelenkimplantat nach einem der vorangehenden Ansprüche, **dadurch gekennzeichnet, dass** der pfannenartige Tragkörper (6) aus einem nicht kaltverformbaren Material besteht.

9. Hüftgelenkimplantat nach Anspruch 8, **dadurch gekennzeichnet, dass** der pfannenartige Tragkörper (6) aus einer Titanlegierung besteht, die weniger verformbar ist als Reintitan, vorzugsweise mit einer Zugfestigkeit von mindestens 800 MPa.

10. Hüftgelenkimplantat nach einem der vorangehenden Ansprüche, **dadurch gekennzeichnet, dass** die Befestigungslaschen (2, 3) und ggf. der Befestigungsring (4) aus Reintitan, vorzugsweise gemäß Grad 2, Grad 3 oder 4, und der pfannenartige Tragkörper (6) aus einer Titanlegierung, insbesondere TiAl6V4, bestehen.

11. Hüftgelenkimplantat nach einem der vorangehenden Ansprüche, **dadurch gekennzeichnet, dass** ein Übergangsbereich zwischen dem pfannenartigen Tragkörper (6) und Befestigungslaschen/-ring (2, 3, 4) mit einer gefrästen Oberfläche versehen ist.

12. Modulares Hüftgelenkssystem mit einem Hüftgelenkimplantat nach einem der vorangehenden Ansprüche, **dadurch gekennzeichnet, dass** das modulare Hüftgelenkssystem einen Befestigungsring (4) umfasst, der ausgebildet ist zur Verbindung mit einer oder mehreren von einer Mehrzahl von pfannenartigen Tragkörpern (6) in verschiedenen Größen.

13. Modulares Hüftgelenkssystem nach Anspruch 12, **dadurch gekennzeichnet, dass** die Mehrzahl von pfannenartigen Tragkörpern (6) Positionierungsnuten (66) aufweisen, die ausgebildet sind zum Positionieren von modularen Lagereinsätzen.

14. Modulares Hüftgelenkssystem nach Anspruch 12, **dadurch gekennzeichnet, dass** jeder der Mehrzahl von pfannenartigen Tragkörpern (6) einen Zentrierring (64) aufweist, wobei der Befestigungsring (4) ausgebildet ist um in jeden Zentrierring (64) von jedem der Mehrzahl von pfannenartigen Tragkörpern (6) zu passen.

15. Modulares Hüftgelenkssystem nach Anspruch 14, **dadurch gekennzeichnet, dass** jeder Zentrierring (64) entweder als ein gesondertes Element oder als eine Ausnehmung ausgeführt ist.
